(19) Europäisches Patentamt
European Patent Office
Office européen des brevets

(11) Veröffentlichungsnummer: **0 130 516**
**B1**

(12) **EUROPÄISCHE PATENTSCHRIFT**

(45) Veröffentlichungstag der Patentschrift: **26.09.90**

(51) Int. Cl.[5]: **A 61 K 31/245,** A 61 K 47/00

(21) Anmeldenummer: **84107249.9**

(22) Anmeldetag: **25.06.84**

# (54) Etofenamat-Zubereitung.

(30) Priorität: **01.07.83 DE 3323832**

(43) Veröffentlichungstag der Anmeldung:
**09.01.85 Patentblatt 85/02**

(45) Bekanntmachung des Hinweises auf die Patenterteilung:
**26.09.90 Patentblatt 90/39**

(84) Benannte Vertragsstaaten:
**AT BE CH DE FR GB IT LI LU NL SE**

(56) Entgegenhaltungen:
**EP-A-0 043 738**
**EP-A-0 054 205**
**EP-A-0 063 870**

**ROTE LISTE, 1982, Seite 452, Editio Cantor, Aulendorf/Württ., DE.**

**Die Akte enthält technische Angaben, die nach dem Eingang der Anmeldung eingereicht wurden und die nicht in dieser Patentschrift enthalten sind.**

(73) Patentinhaber: **Troponwerke GmbH & Co. KG**
**Berliner Strasse 156**
**D-5000 Köln 80 (DE)**

(72) Erfinder: **Dell, Hans-Dieter, Dr.**
**Kalmuentener Strasse 5**
**D-5060 Bergisch-Gladbach 2 (DE)**
Erfinder: **Kraus, Reinhold**
**Paffendorfstrasse 77**
**D-5000 Koeln 91 (DE)**
Erfinder: **Schierstedt, Detlef, Dr.**
**Henri-Dumont-Strasse 2**
**D-5205 St. Augustin 3 (DE)**

(74) Vertreter: **Adrian, Albert, Dr. et al**
**c/o BAYER AG Konzernverwaltung RP**
**Patentabteilung**
**D-5090 Leverkusen 1, Bayerwerk (DE)**

Anmerkung: Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

Courier Press, Leamington Spa, England.

EP 0 130 516 B1

**Beschreibung**

Die vorliegende Erfindung betrifft eine topisch anzuwendende, entzündungshemmende und analgetisch wirkende Arzneimittelzubereitung, welche als Wirkstoff 2-(2-Hydroxyethoxy)-ethyl-N-(α,α,α-trifluor-m-tolyl)anthranilat, nachfolgend Etofenamat enthält. Etofenamat besitzt folgende Struktur:

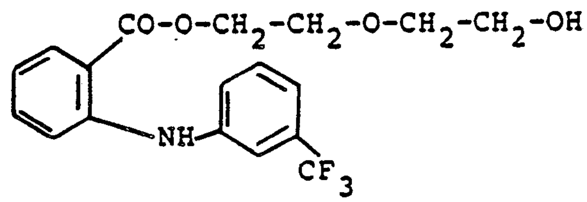

Etofenamat ist bereits als nichtsteroidales Arzneimittel mit guter entzüdungshemmender Wirkung und hervorragender Verträglichkeit bekannt, vgl. Arzneimittelforschung 27 (I), Sonderheft 6b, 1299—1364, 1977. Etofenamat wird bisher in Form alkoholhaltiger Gele eingesetzt. Durch den Alkohol wirkt das Gel beim Auftragen kühlend, was nicht von allen Patienten als angenehm empfunden wird. Ferner kann ein solches Gel nur bei unversehrter Haut aufgetragen werden, eine wünschenswerte Applikation bei Traumen mit offenen Wunden entfällt daher. Auch ist bei Patienten mit ausgesprochen trockener Haut eine solche Formulierung nur bedingt geeignet.

Eine Aufgabe der vorliegenden Erfindung ist es daher, die geschilderten Nachteile durch Bereitstellung einer neuen Zubereitung zu überwinden, welche jedoch die entzündungshemmende und analgetische Wirkung im gleichen Ausmaß wie die Gelform aufweist.

Überraschenderweise ist es gelungen, eine Creme-Zubereitung zu finden, welche die geforderten Eigenschaften aufweist.

Gegenstand der vorliegenden Erfindung ist daher eine Creme-Zubereitung enthaltend 2—30 Gew.-% Etofenamat sowie 5—80 Gew.-% Isopropylmyristat als Adjuvans.

Etofenamat stellt ein hochviskoses Öl dar, welches in Wasser nur in Spuren löslich ist. In einer Vielzahl von galenischen Hilfsstoffen besteht nur eine mangelnde Löslichkeit, finden Interaktionen, wie z.B. Hydrolyse, Umesterung, Veresterung etc. statt, tritt Entmischung, Phasentrennung etc. auf. Einige Formulierungen mit Etofenamat haben sich zwar als homogen und stabil erwiesen, weisen jedoch eine im Vergleich zum bekannten Etofenamat-haltigen Gel ungenügende Resorption auf. Diese Nachteile treten bei der erfindungsgemäßen Zubereitung nicht auf. In der erfindungsgemäßen Zubereitung wird Etofenamat hervorragend von der Haut aufgenommen.

Die erfindungsgemäße Creme-Zubereitung enthält daher neben Etofenamat und Adjuvans gegebenenfalls Konsistenzgeber und/oder Emulgatoren sowie weitere Hilfsstoffe, wie durchblutungsfördernde bzw. wärmende oder angenehm riechende Zusätze.

Ausgehend von diesen Etofenamat-Adjuvans-Gemischen bzw. Lösungen können verschiedene Cremes der Konsistenz flüssig bis halbfest hergestellt werden:

1. Öl in Wasser-Emulsionen
2. Wasser in Öl-Emulsionen und
3. Mikroemulsionen.

Erfindungsgemäß ist es notwendig, daß die aus Wirkstoff und Adjuvans bestehende Creme als Wirkstoff ca. 2 bis ca. 30 Gew.-% Etofenamat und ca. 5 bis ca. 80 Gew.-% Isopropylmyristat als Adjuvans enthält. Bevorzugt enthält die Creme-Zubereitung 5—15 Gew.-% Etofenamat, besonders bevorzugt 6—12 Gew.-%

Ferner kann die Creme-Zubereitung noch einen oder mehrere Konsistenzgeber und/oder einen oder mehrere Emulgatoren und/oder durchblutungsfördernde bzw. wärmende Zusätze und/oder weitere Hilfsstoffe, wie z.B. angenehm riechende Stoffe und/oder auch weitere Wirkstoffe enthalten.

In vielen Fällen sind auch Konsistenzgeber und Emulgatoren identisch, eine strikte Trennung ist daher nicht möglich.

Als Konsistenzgeber kann einer oder mehrere aus der Gruppe: Fettalkohole der Kettenlänge $C_{10}$ bis $C_{30}$, wie z.B. Stearylalkohol, Kohlenwasserstoffe, wie Vaseline, Paraffine, Hartparaffine, Wachse wie Bienen- oder Carnaubawachs eingesetzt werden. Außerdem können noch Mono-, Di- und Triglyceride wie Glycerinmonostearat, Glycerindistearat, Metallseifen wie Al-stearat, Aerosil, Polyglykole wie Polyethylenglykole, Polypropylenglykole verwendent werden.

Die Konsistenz der wäßrigen Phase von wasserhaltigen Creme-Zubereitungen kann durch Hydrogelbildner wie Veegum, Cellulosederivate (z.B. Methylcellulose, Hydroxyethylcellulose, Carboxymethylcellulose), Carboxyvinylpolymere, Alginsäurederivate, Gummi arabicum usw. eingestellt werden.

Als Emulgator kann einer oder mehrere aus der Gruppe der ionogenen oder nichtionogenen Wasserin-Öl und Öl in Wasser Emulgatoren eingesetzt werden.

Bevorzugt werden Salze höherer Fett- und Gallensäuren wie Triethanolaminstearat, Alkylsulfate wie

Na-Laurylsulfat, Alkylsulfonate wie N-Cetylsulfonat, höhere Fettalkohole wie Cetylalkohol, Laurylkalkohol und Sterylalkohol, Sterinalkohole wie Cholesterin, Fettsäureester mehrwertiger Alkohole und Polyole wie Ethylenmonostearrat, Glycerinmonooleat, Sorbitanmonolaurat, Sorbitantrioleat, Polyoxyethylen-(20) sorbitanmonolaurat, Polyoxyethylen-Sorbit-Hexaoleat, Fettalkoholether wie Polyoxyethylenlaurylether, Polyoxyethylenoleylether, Fettsäureester der Saccharose wie Saccharosedistearat, Lecithin und Derivate, Betaine und Sulfobetaine wie Fettsaüreamidoalkylbetain und/oder Polyoxyethylenpolyoxypropylen-Polymere wie Pluronics eingesetzt.

Als durchblutungsfördende oder wärmende Zusätze können z.B. Benzylnicotinat, Salicylsäureester, z.B. Methylsalicylat, etherische Öle, Capsaicin, Capsicumextrakt oder Nonylsäurevanillylamid eingesetzt werden.

Als Geruchsstoffe können die üblichen pharmakologisch unbedenklichen Stoffe eingesetzt werden, sofern sie sich mit dem Wirkstoff und Adjuvans vertragen.

Die Menge der Konsistenzgeber und/oder Emulgatoren in der Creme-Zubereitung beträgt neben Etofenamat und Isopropylmyristat als Adjuvans bis zu ca. 70 Gew.-%, bevorzugt 5—25 Gew.-%.

Ferner enthält die Creme-Zubereitung noch Wasser bis zu 85 Gew.-%, bevorzugt 40—60 Gew.-%.

Besonders bevorzugt sind Creme-Zubereitungen, die 2—30 Gew.-% Etofenamat, 5—80 Gew.-% Isopropylmyristat als Adjuvans und 40—60 Gew.-% Wasser enthalten.

Besonders bevorzugt sind ferner die Zubereitungen, deren Zusammensetzung aus den Beispielen hervorgeht.

Erfindungsgemäß werden die erfindungsgemäßen entzündungshemmenden, analgetischen Creme-Zubereitungen hergestellt, indem das in einem geeigneten Lösungsmittel gelöste Etofenamat mit Isopropylmyristat und gegebenenfalls übrigen aufgeschmolzenen Fett-/Wachs-Emulgator-Bestandteilen gemischt und anschließend die auf etwa gleiche Temperatur gebrachte wäßrige Phase, welche Konservierungsmittel, Puffer, etc. enthält, unter intensivem Mischen der Bestandteile hinzugegeben wird. Anschließend erfolgt eine Homogenisierung, beispielsweise mittels einer Rotor-Stator-Einrichtung.

Die Homogenisierungstemperatur ist von der jeweiligen Zubereitung abhängig und liegt im allgemeinen zwischen ca. 40°C und ca. 60°C.

In der erfindungsgemäßen Creme-Zubereitung des Etofenamats ist der Wirkstoff in stabiler Form enthalten und vermag ohne die Schlepperfunktion von z.B. Isopropanol die Haut zu penetrieren. Vergleichsstudien zu Etofenamat Gel ergeben bioäquivalente Resorption, was durch annähernd gleiche Resorptionsgeschwindigkeit, Zeit maximalen Plasmaspiegels, maximaler Plasmaspiegelkonzentration, Elimination aus dem Plasma, dem Flächeninhalt unter den Plasmaspielgekurven (AUC) sowie der renalen Elimination beim Menschen belegt werden konnte.

Die Gleichwertigkeit der Resorption ergibt sich auch aus klinischen Prüfungen, in denen die Wirksamkeit im Doppelblind cross over-Versuch gegen 1-(p-Chlorbenzoyl)-5-methoxy-2-methylindol-3-essigsäure enthaltende Salbe sowie gegen Salicylat-haltige Salben verglichen wurde und bei denen die erfindungsgemäße Creme-Zubereitung z.B. bei Lumbago hinsichtlich Schober-Distanz, Spontan-, Bewegungs- und Druckschmerz gegenüber dem ersten Vergleichspräparat signifikant ($p < 0{,}05$) besser wirkte und sich z.B. bei akuter Gonathrose bei den Zielgrößen Gelenkbeweglichkeit, Gelenkumfang, Spontan- und Bewegungsschmerz gegenüber dem zweiten Vergleichspräparat signifikant wirksamer erwies.

Die vorliegende Erfindung soll durch die nachfolgenden Beispiele noch weiter veranschaulicht werden.

| Beispiel | 1 | 2 | 3 | 4. |
|---|---|---|---|---|
| Etofenamat (mg) | 100 | 100 | 200 | 50 |
| $H_2O$ (mg) | 522,5 | 512,5 | 442 | 582 |
| Adjuvans (mg) | Isopropyl-myristat 100 | Isopropyl-myristat 100 | Isorpopyl-myristat 100 | Isopropyl-myristat 100 |
| Emulgator (mg) | Myrj. 59 50 | Myrj. 59 50 | Myrj. 59 50 | Myrj. 59 50 |
| Benzylkalkohol (mg) | 15 | 15 | 15 | 15 |
| Tylose MH 4000 (mg) | 7,5 | 7,5 | 8,0 | 8,0 |
| Konsistenzgeber (mg) | Cutina MD 175 | Cutina MD 175 | Cutina MD 155 | Cutina MD 165 |
| Citratpufferlösung (mg) | 30 | 30 | 30 | 30 |
| Nicotinsäurebenzylester | — | 10 | — | — |

| | | |
|---|---|---|
| Miglyol 840 | = | Propylenglykoldiester der Capryl-Caprinsäure |
| Myrj 59 | = | Polyoxyethylenfettsäureester |
| Twun 40 | = | Polyoxyethylen(20)sorbitanmonopalmitat |
| Twun 60 | = | Polyoxyethylen(20)sorbitanmonopalmitat |
| Cremophor El | = | Glycerin-Polyethylenglykolricinoleat |
| Cutina MD/GMS | = | Gemisch von Mono- und Diglyceriden der Palmitrin- und Stearinsäure |
| Softisan 378 | = | Triglyceride der Stearin-, Caprin- und Caprylsäure |

**Patentansprüche**

1. Cremezubereitung für Etofenamat, dadurch gekennzeichnet, daß sie 5 bis 80 Gew.-% Isopropylmyristat und 2 bis 30 Gew.-% Etofenamat enthält.

2. Verfahren zur Herstellung von Cremezubereitungen nach Anspruch 1, dadurch gekennzeichnet, daß man in einem geeigneten Lösungsmittel gelöstes Etofenamat mit Isopropylmyristat und gegebenenfalls übrigen Hilfsstoffen mischt, die wäßrige Phase unter intensivem Vermischen zugibt und anschließend die Mischung homogenisiert.

**Revendications**

1. Composition en crème à base d'Etofenamat, caractérisée en ce qu'elle contient de 5 à 80% en poids de myristate d'isopropyle et de 2 à 30% en poids d'Etofenamat.

2. Procédé de préparation de la composition en crème selon la revendication 1, caractérisé en ce que l'on mélange l'Etofenamat, dissous dans un solvant approprié, avec le myristate d'isopropyle et le cas échéant d'autres produits auxiliaires, on ajoute la phase aqueuse sous mélange énergique et on homogénéise ensuite le mélange.

**Claims**

1. Cream formulation for etofenamate, characterized in that it contains 5 to 80% by weight of isopropyl myristate and 2 to 30% by weight of etofenamate.

2. Process for the preparation of cream formulations according to Claim 1, characterized in that etofenamate, dissolved in a suitable solvent, is mixed with isopropyl myristate and, if appropriate, other auxiliaries, the aqueous phase is added, with intensive mixing, and the mixture is then homogenised.